(19) Europäisches Patentamt
European Patent Office
Office européen des brevets

(11) **EP 3 235 902 A1**

(12) **EUROPEAN PATENT APPLICATION**
published in accordance with Art. 153(4) EPC

(43) Date of publication:
**25.10.2017 Bulletin 2017/43**

(21) Application number: **15867833.4**

(22) Date of filing: **10.12.2015**

(51) Int Cl.:
*C12N 5/071* $^{(2010.01)}$    *C12M 1/00* $^{(2006.01)}$
*C12M 3/00* $^{(2006.01)}$

(86) International application number:
**PCT/JP2015/084698**

(87) International publication number:
**WO 2016/093321 (16.06.2016 Gazette 2016/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **11.12.2014 JP 2014250958**

(71) Applicants:
• **Icomes Lab Co., Ltd.**
**Iwate 020-0857 (JP)**
• **National Institute of Advanced Industrial Science
and Technology**
**Tokyo 100-8921 (JP)**

(72) Inventors:
• **SUZUKI, Jun**
**Morioka-shi**
**Iwate 020-0857 (JP)**
• **KAMIYAMA, Tadataka**
**Morioka-shi**
**Iwate 020-0857 (JP)**
• **OKONOGI, Atsuhito**
**Morioka-shi**
**Iwate 020-0857 (JP)**
• **NATSUME, Toru**
**Tokyo 135-0064 (JP)**

(74) Representative: **Hoffmann Eitle**
**Patent- und Rechtsanwälte PartmbB**
**Arabellastraße 30**
**81925 München (DE)**

(54) **CELL CULTURE METHOD AND CELL CULTURE DEVICE**

(57)    It is an object of the present invention to provide a cell culture method and a cell culture apparatus capable of culturing cells for a long period of time without causing stress or damage to the cells. Therefore, the cell culture apparatus includes a supply port 4 provided at one end of a culture dish 3, a discharge port 5 provided at the other end of the culture dish 3 so as to sandwich cultured cells 6 between the supply port 4 and the discharge port 5, a reservoir tank 11 that stores a culture liquid to be supplied to the culture dish 3, a waste liquid tank 21 that stores the culture liquid to be discharged from the culture dish, a supply-side micro flow rate pump 12 that supplies the culture liquid in the reservoir tank 11 to the culture dish 3 through the supply port 4, a discharge-side micro flow rate pump 22 that discharges the culture liquid from the culture dish 3 through the discharge port 5, and a flow rate controller 30 that performs flow rate control of the supply-side micro flow rate pump 12 and the discharge-side micro flow rate pump 22 so that a moving linear velocity of the culture liquid from the supply port 4 toward the discharge port 5 is less than a maximum velocity at which shear stress is not applied to the cultured cells.

FIG.2

## Description

Field

The present invention relates to a cell culture method and a cell culture apparatus capable of culturing cells for a long period of time without causing stress or damage to the cells.

Background

As regenerative medicine using stem cells in recent years, for example, treatment of liver cirrhosis, blood disease, and myocardial infraction, construction of blood vessels, regeneration of bones and cornea, securing skin for transplantation are conceivable. In regenerative medicine, desired cells and organs are expanded from stem cells and the like in a culture dish so as to be transplanted to a person. Recently, angiogenesis is performed by stem cells derived from bone marrow, and treatment for angina pectoris, myocardial infarction, etc. is successfully performed.

Here, in a conventional cell culture apparatus, a culture liquid in a culture dish is periodically exchanged to grow cultured cells. The cell culture apparatus has a problem that the cells are stressed or damaged because the cells are greatly stimulated in association with exchange of the culture liquid and a waste product is discharged into the culture liquid in association with the metabolic activity of the cells.

On the other hand, there is a device that carries out cell culture by feeding a culture liquid to a culture dish without exchanging the culture liquid. For example, Patent Literature 1 describes a cell culture apparatus that monitors the growth rate of cells, predicts a decrease in nutrients in a culture liquid based on the monitored growth rate, and adds a consumed amount of nutrients to the culture liquid.

In addition, there is a method in which cell culture is carried out by feeding and discharging a culture liquid without exchanging the culture liquid. For example, Patent Literature 2 describes a cell culture method in which a culture liquid is circulated in a culture tank by a filter module made of hollow fibers to add nutrients in the culture liquid and remove waste product.

Citation List

Patent Literature

Patent Literature 1: Japanese Laid-open Patent Publication No. 2012-170366
Patent Literature 2: Japanese Laid-open Patent Publication No. 2012-090632

Summary

Technical Problem

However, because the cell culture apparatus described in Patent Literature 1 adopts a system of collecting the culture liquid in a culture tank although the nutrients are continuously fed to the culture tank, when the concentration of the waste product discharged by metabolic activity of cells in the culture liquid increases, the culture liquid in the culture tank has to be exchanged at one time at arbitrary timing similarly to the conventional cell culture apparatus. Frequency of exchanging all the culture liquid is once in a day, which results in bringing stress or damage to the cells similarly to the conventional cell culture apparatus.

On the other hand, in Patent Literature 2, it is configured to continuously feed and discharge an extremely small amount of culture liquid, however, when a culture period is long, the filter module is clogged due to the waste product, so that the method is not suitable for long term culture. Particularly because the culture period until cells required for recent regenerative medicine or the like are obtained is long such as about one month, the failure of long term culture means that cells having a large cell area required for regenerative medicine etc. cannot be cultured.

The present invention has been made to solve the conventional problems, and it is an object of the present invention to provide a cell culture method and a cell culture apparatus capable of culturing cells for a long period of time without causing stress or damage to the cells.

Solution to Problem

To solve the problem described above and to achieve the object, a cell culture method according to the present invention is a cell culture method for arranging cultured cells in a culture dish and continuously culturing the cultured cells by supplying a liquid required to grow or maintain the cultured cells to the culture dish and discharging the liquid from the culture dish. The cell culture method includes: providing a supply port of the liquid at one end of the culture dish and providing a discharge port of the liquid at other end of the culture dish so as to sandwich the cultured cells between the supply port and the discharge port, and discharging the liquid while supplying the liquid to the culture dish so that a moving linear velocity of the liquid from the supply port toward the discharge port is less than a maximum velocity at which shear stress is not applied to the cultured cells.

In the cell culture method according to the present invention, the moving linear velocity of the liquid is equal to or less than a diffusion velocity due to molecular motion of the liquid.

A cell culture apparatus according to the present invention is a cell culture apparatus configured to arrange cultured cells in a culture dish and continuously culture the cultured cells by supplying a liquid required to grow or maintain the cultured cells to the culture dish and discharging the liquid from the culture dish. The cell culture apparatus includes: a supply port of the liquid provided at one end of the culture dish; a discharge port for the culture dish provided at other end of the culture dish so as to sandwich the cultured cells between the supply port and the discharge port, a reservoir tank configured to store the liquid to be supplied to the culture dish; a waste liquid tank configured to store the liquid to be discharged from the culture dish; a supply-side micro flow rate pump configured to supply the liquid in the reservoir tank to the culture dish through the supply port; a discharge-side micro flow rate pump configured to discharge the liquid from the culture dish through the discharge port; and a flow rate controller configured to perform flow rate control of the supply-side micro flow rate pump and the discharge-side micro flow rate pump so that a moving linear velocity of the liquid from the supply port toward the discharge port is less than a maximum velocity at which shear stress is not applied to the cultured cells.

In the cell culture apparatus according to the present invention, the moving linear velocity of the liquid is equal to or less than a diffusion velocity due to molecular motion of the liquid.

In the cell culture apparatus according to the present invention, a side face of the culture dish has surface free energy smaller than surface free energy of a bottom face of the culture dish.

The cell culture apparatus according to the present invention further includes: a horizontal adjustment mechanism configured to adjust the bottom face of the culture dish horizontally.

The cell culture apparatus according to the present invention further includes: an inclination adjustment mechanism configured to adjust the bottom face of the culture dish diagonally.

In the cell culture apparatus according to the present invention, the supply port includes a plurality of supply ports which are discretely arranged in linear order, and the discharge port includes a plurality of discharge ports which are discretely arranged in linear order.

In the cell culture apparatus according to the present invention, an outlet of the supply-side micro flow rate pump and the supply port are connected by a flexible tube, and the discharge port and an inlet of the discharge-side micro flow rate pump are connected by a flexible tube, and a diaphragm adjustment mechanism configured to adjust an opening of the flexible tube connected between the outlet of the supply-side micro flow rate pump and the supply port is provided between the supply-side micro flow rate pump and the supply port, and a diaphragm adjustment mechanism configured to adjust an opening of the flexible tube connected between the discharge port and the inlet of the discharge-side micro flow rate pump is provided between the discharge-side micro flow rate pump and the discharge port.

In the cell culture apparatus according to the present invention, the reservoir tank and an inlet of the supply-side micro flow rate pump are detachably and directly connected to each other, an outlet of the supply-side micro flow rate pump and the supply port are detachably and directly connected to each other, the discharge port and an inlet of the discharge-side micro flow rate pump are detachably and directly connected to each other, and an outlet of the discharge-side micro flow rate pump and the waste liquid tank are detachably and directly connected to each other.

In the cell culture apparatus according to the present invention, a multistage configuration in which the reservoir tank is provided on an upper side of a supply-side flow path that connects between the supply port and the outlet of the supply-side micro flow rate pump is disposed horizontally in a manner that one end of the multistage configuration provided the reservoir tank therein is directed to the supply port of the culture dish, and at other end of the multistage configuration provided the reservoir tank therein, the inlet of the supply-side micro flow rate pump is detachably connected to the reservoir tank and the outlet of the supply-side micro flow rate pump is detachably connected to an inlet of the supply-side flow path, and a multistage configuration in which the waste liquid tank is provided on an upper side of a discharge-side flow path that connects between the discharge port and the inlet of the discharge-side micro flow rate pump is disposed horizontally in a manner that one end of the multistage configuration provided the waste liquid tank therein is directed to the discharge port of the culture dish, and at the other end of the multistage configuration provided the waste liquid tank therein, the outlet of the discharge-side micro flow rate pump is detachably connected to the waste liquid tank and the inlet of the discharge-side micro flow rate pump is detachably connected to an outlet of the discharge-side flow path.

The cell culture apparatus according to the present invention further includes: a flow path plate in which a first flow path between the reservoir tank and the supply-side micro flow rate pump, a second flow path between the supply-side micro flow rate pump and the culture dish, a third flow path between the culture dish and the discharge-side micro flow rate pump, and a fourth flow path between the discharge-side micro flow rate pump and the waste liquid tank are embedded, the flow path plate being disposed on an upper side of the reservoir tank, the culture dish, and the waste liquid tank. The first flow path and an inlet of the supply-side micro flow rate pump are pin port connected to each other, the second flow path and an outlet of the supply-side micro flow rate pump are pin port connected to each other, the third flow path and an inlet of the discharge-side micro flow rate pump are pin port connected to each other, and the fourth flow path and an outlet pipe of the supply-side micro flow rate pump are pin port connected to each other.

The cell culture apparatus according to the present invention further includes: a liquid level detection sensor configured to detect a liquid level in the culture dish. The flow rate controller is configured to perform flow rate control so that the liquid level detected by the liquid level detection sensor becomes constant.

In the cell culture apparatus according to the present invention, a supply-side drive source for driving the supply-side micro flow rate pump is configured to be detachably attached to the supply-side micro flow rate pump, and a discharge-side drive source for driving the discharge-side micro flow rate pump is configured to be detachably attached to the discharge-side micro flow rate pump.

The cell culture apparatus according to the present invention further includes: a substrate configured to arrange at least the culture dish. The substrate has a hole or a colorless and transparent concave portion in an area of the substrate where a culture state of the cultured cells is observed, the concave portion having an opening formed in the substrate on a side opposite to a side of the substrate on which the culture dish is arranged.

The cell culture apparatus according to the present invention further includes: a transparent conductive film heater provided at a bottom face of the culture dish; a temperature sensor configured to detect a temperature of the liquid in the culture dish; and a temperature controller configured to perform control to keep the temperature of the liquid in the culture dish within a predetermined temperature range by energizing the transparent conductive film heater based on detection result of the temperature sensor.

Advantageous Effects of Invention

According to the present invention, the supply port of the liquid required to grow or maintain cultured cells is provided at one end of the culture dish and the discharge port of the liquid is provided at the other end of the culture dish so that the cultured cells are sandwiched between the supply port and the discharge port, and the liquid is discharged while being supplied to the culture dish so that the moving linear velocity of the liquid from the supply port toward the discharge port is less than the maximum velocity at which shear stress is not applied to the cultured cells. Therefore, it is possible to culture the cells for a long period of time without causing stress or damage to the cells and suppress consumption of the liquid.

Brief Description of Drawings

FIG. 1 is a perspective view illustrating an appearance configuration of a cell culture apparatus according to an embodiment of the present invention.
FIG. 2 is a block diagram illustrating a configuration of the cell culture apparatus illustrated in FIG. 1.
FIG. 3 is a cross-sectional view illustrating a configuration of a hole made on a substrate at an area where a culture dish is provided.
FIG. 4 is a cross-sectional view illustrating a configuration of a hole made on a substrate at an area where a reservoir tank is provided.
FIG. 5 is an explanatory diagram for explaining a function of a diaphragm adjustment mechanism.
FIG. 6 is a diagram illustrating an incidence rate of cells affected by shear stress appearing in cultured cells with respect to a moving linear velocity of a culture liquid from a supply port toward a discharge port.
FIG. 7 is a diagram illustrating a state image of the culture liquid flowing from a supply-port side face toward a discharge-port side face.
FIG. 8 is a diagram illustrating movement of the culture liquid with a passage of time as a change in relative concentration.
FIG. 9 is a flowchart illustrating a procedure for controlling a flow rate of the culture liquid by a flow rate controller.
FIG. 10 is a diagram illustrating an example of an arrangement configuration of supply port openings.
FIG. 11 is a diagram illustrating an example of an arrangement configuration of supply port openings.
FIG. 12 is a diagram illustrating a state image of the culture liquid flowing from the supply-port side face toward the discharge-port side face when a fluorine-based water repellent agent is applied to a side face of the culture dish.
FIG. 13 is a front view illustrating a configuration near the culture dish of the cell culture apparatus provided with a horizontal adjustment mechanism.
FIG. 14 is a partially broken front view illustrating a specific configuration of the horizontal adjustment mechanism.
FIG. 15 is a diagram illustrating an example of an arrangement configuration and a connection configuration of a liquid feeding portion and a liquid discharging portion.
FIG. 16 is a diagram illustrating an example of an arrangement configuration and a connection configuration of the liquid feeding portion and the liquid discharging portion.
FIG. 17 is a perspective view illustrating an arrangement configuration of cell culture apparatuses in a transportation box.
FIG. 18 is a plan view illustrating a configuration of a cell culture apparatus using a closed perfusion system flow path.
FIG. 19 is an A-A line cross-sectional view of the cell culture apparatus illustrated in FIG. 18.

Description of Embodiments

Some embodiments for implementing the present invention will be explained below with reference to the accompanying drawings.

Overall Configuration

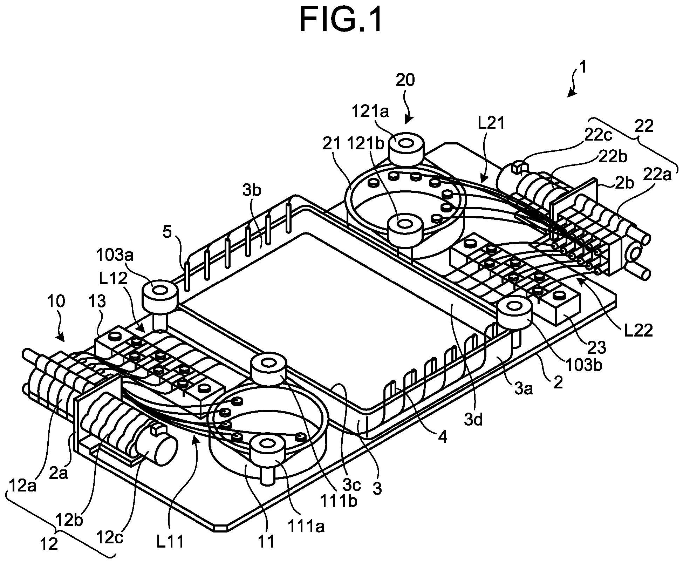

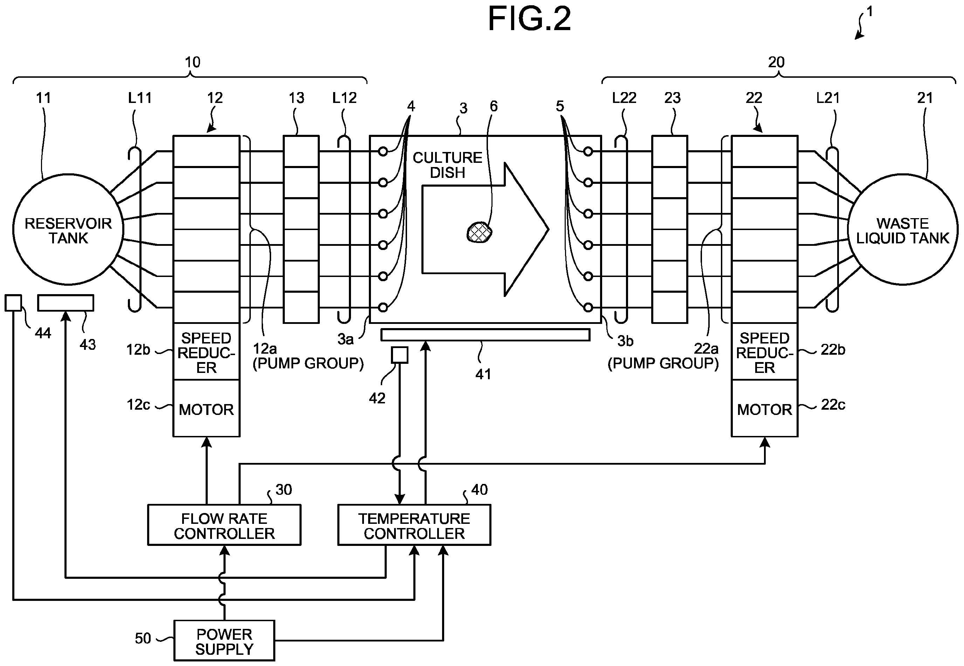

FIG. 1 is a perspective view illustrating an appearance configuration of a cell culture apparatus 1 according to an embodiment of the present invention. FIG. 2 is a block diagram illustrating a configuration of the cell culture apparatus 1 illustrated in FIG. 1. As illustrated in FIG. 1 and FIG. 2, the cell culture apparatus 1 includes a culture dish 3 provided at the center of the upper side of a substrate 2, and also includes a liquid feeding portion 10 for supplying a culture liquid to the culture dish 3 and a liquid discharging portion 20 for discharging the culture liquid from the culture dish 3 which are provided on the substrate 2 so as to sandwich the culture dish 3 from both ends of the culture dish 3.

In the culture dish 3, a supply port 4 of the culture liquid is provided on a supply-port side face 3a, which is one end side of the culture dish 3, and a discharge port 5 of the culture liquid is provided on a discharge-port side face 3b, which is the other end side of the culture dish 3 so as to sandwich cultured cells 6 between the supply port 4 and the discharge port 5. Six supply ports 4 and six discharge ports 5 are provided, and the supply ports and the discharge ports are discretely arranged linearly along the supply-port side face 3a and the discharge-port side face 3b, respectively. The openings of the supply port 4 and the discharge port 5 are provided at positions at any depth less than the depth of the culture liquid in the culture dish 3.

The liquid feeding portion 10 includes a reservoir tank 11, a supply-side micro flow rate pump 12, and a diaphragm adjustment mechanism 13. The reservoir tank 11 stores the culture liquid. The supply-side micro flow rate pump 12 supplies the culture liquid in the reservoir tank 11 to the supply port 4 of the culture dish 3. The diaphragm adjustment mechanism 13 has a variable diaphragm function of readjusting the flow rate of the culture liquid supplied from the supply-side micro flow rate pump 12.

A culture liquid outlet of the reservoir tank 11 and a culture liquid inlet of the supply-side micro flow rate pump 12 are connected by a flow path L11 including six flexible tubes. Moreover, a culture liquid outlet of the supply-side micro flow rate pump 12 and the supply port 4 are connected by a flow path L12 including six flexible tubes. The diaphragm adjustment mechanism 13 can perform variable diaphragm on each of the six flexible tubes of the flow path L12.

The supply-side micro flow rate pump 12 has a pump group 12a including six peristaltic pumps, a speed reducer 12b, and a motor 12c. Each peristaltic pump can be connected in multiple stages along its rotation axis. The speed reducer 12b reduces the rotation of the motor 12c in multiple stages and transmits the reduced rotation to the pump group 12a. The motor 12c can be attached to and detached from the speed reducer 12b. A flange 2a erected on the substrate 2 is formed on the substrate 2 on the liquid feeding portion 10 side. The pump group 12a is attached to one end face side of the flange 2a and the speed reducer 12b is attached to the other end face side of the flange 2a, so that the speed reducer 12b and the pump group 12a are connected to each other. The speed reducer 12b is configured to be detachably attached to the flange 2a and the pump group 12a.

On the other hand, the liquid discharging portion 20 includes a waste liquid tank 21, a discharge-side micro flow rate pump 22, and a diaphragm adjustment mechanism 23. The discharge-side micro flow rate pump 22 discharges the culture liquid in the culture dish from the discharge port 5 of the culture dish 3. The waste liquid tank 21 stores the culture liquid discharged by the discharge-side micro flow rate pump 22. The diaphragm adjustment mechanism 23 has a variable diaphragm function of readjusting the flow rate of the culture liquid discharged from the discharge-side micro flow rate pump 22.

The discharge port 5 and a culture liquid inlet of the discharge-side micro flow rate pump 22 are connected by a flow path L22 including six flexible tubes. Moreover, a culture liquid outlet of the discharge-side micro flow rate pump 22 and a culture liquid inlet of the waste liquid tank 21 are connected by a flow path L21 including six flexible tubes. The diaphragm adjustment mechanism 23 can perform variable diaphragm on each of the six flexible tubes of the flow path L22.

The discharge-side micro flow rate pump 22 has a pump group 22a including six peristaltic pumps, a speed reducer 22b, and a motor 22c. Each peristaltic pump can be connected in multiple stages along its rotation axis. The speed reducer 22b reduces the rotation of the motor 22c in multiple stages and transmits the reduced rotation to the pump group 22a. The motor 22c can be attached to and detached from the speed reducer 22b. A flange 2b erected on the substrate 2 is formed on the substrate 2 on the liquid discharging portion 20 side. The pump group 22a is attached to one end face side of the flange 2b and the speed reducer 22b is attached to the other end face side of the flange 2b, so that the speed reducer 22b and the pump group 22a are connected to each other. The speed reducer 22b is configured to be detachably attached to the flange 2b and the pump group 22a.

As illustrated in FIG. 1, the culture dish 3 is attached to the substrate 2 by holding members 103a and 103b. The reservoir tank 11 is attached to the substrate 2 by holding members 111a and 111b. The waste liquid tank 21 is attached to the substrate 2 by holding members 121a and 121b.

As explained above, the motors 12c and 22c and the speed reducers 12b and 22b are removable and can be removed upon autoclaving. Because the culture dish 3 is usually formed of polycarbonate, it is exchanged each time the autoclaving is performed.

As illustrated in FIG. 2, the motor 12c and the motor 22c are driven by a flow rate controller 30. The pump groups 12a and 22a control the flow rate of the culture liquid using the flow rate controller 30. A transparent conductive film heater 41 and a temperature sensor 42 are provided at the bottom face of the culture dish 3. A Peltier element 43 and a temperature sensor 44 are provided in the reservoir tank 11. A temperature controller 40 controls temperature so that a temperature of the culture liquid in the culture dish 3 will reach a desired temperature, for example, 37°C, by controlling energization of the transparent conductive film heater 41 based on the detection result of the temperature sensor 42 and controls temperature so that a temperature of the culture liquid in the reservoir tank 11 will reach a desired temperature, for example, 5 to 20°C, by controlling energization of the Peltier element 43 based on the detection result of the temperature sensor 44. A power supply 50 supplies power to the flow rate controller 30 and to the temperature controller 40. The culture liquid in the culture dish 3 needs to be set to a temperature suitable for growth of cells, and the culture liquid in the reservoir tank 11 needs to be set to a temperature at which it can be stored for a long time. Although the culture liquid in the reservoir tank 11 is set to a temperature lower than that of the culture liquid in the culture dish 3, the culture liquid flowing out of the reservoir tank 11 has a temperature close to ordinary temperature by passing through the flow paths L11 and L12 until it reaches the supply port of the culture dish 3.

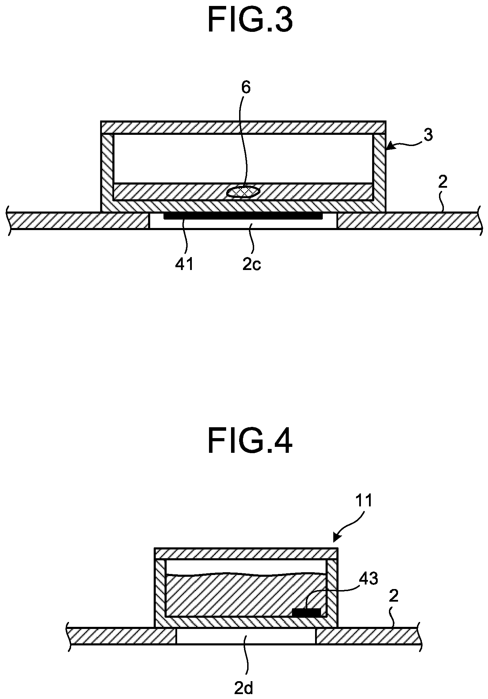

As illustrated in FIG. 3, the substrate 2 includes a hole 2c such as a circular hole, which is provided in an area of the substrate 2 where the culture dish 3 is disposed. The reason for providing the hole 2c is to shorten the length of the culture dish 3 in the height direction so that the state of the cell during cell culture can be observed by an optical microscope. Therefore, the substrate 2 may include a concave portion hollowed out a lower portion of the substrate 2 at a position where the culture dish 3 is disposed. However, the bottom face of the concave portion is preferably colorless and transparent.

As illustrated in FIG. 4, the substrate 2 includes a hole 2d such as a circular hole, which is provided in an area of the substrate 2 where the reservoir tank 11 is disposed. This is because the state of the culture liquid in the reservoir tank 11 can be visually observed through the hole 2d. When the state of the culture liquid in the reservoir tank 11 deteriorates, turbidity and discoloration occur. Similarly, it is preferable that the substrate 2 includes a hole provided in an area of the substrate 2 where the waste liquid tank 21 is disposed.

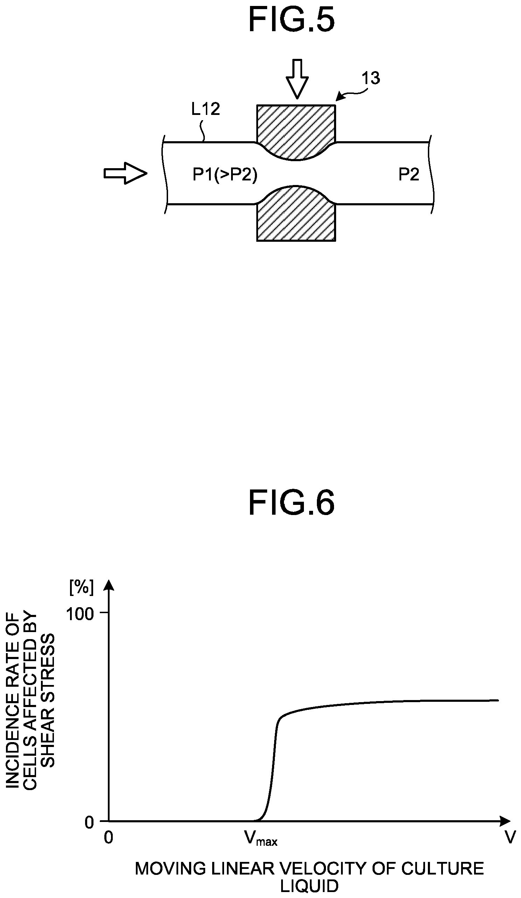

As illustrated in FIG. 5, the diaphragm adjustment mechanism 13 can form a diaphragm for narrowing the flow path opening by pressing the flexible tube which is the flow path L12 so as to be held. As explained above, the diaphragm adjustment mechanism 13 discretely readjusts the flow rate of each flexible tube of the flow path L12. By forming the diaphragm in the flow path L12, pressure P1 on an upstream side of the diaphragm becomes larger than pressure P2 on a downstream side thereof. As a result, bubbles or the like are less likely to be generated in the culture liquid when the pump group 12a arranged on the upstream side of the diaphragm sucks and discharges the culture liquid, thus accurately controlling the flow rate.

Flow of Culture Liquid in Culture Dish

Here, in the present embodiment, it is configured so that the culture liquid is discharged while being supplied to the culture dish 3 so that the moving linear velocity V of the culture liquid from the supply port 4 toward the discharge port 5 in the culture dish 3 is less than the maximum velocity at which shear stress is not applied to the cultured cells 6.

FIG. 6 is a diagram illustrating an incidence rate of cells affected by shear stress appearing in the cultured cells 6 with respect to the moving linear velocity V of the culture liquid from the supply port 4 toward the discharge port 5. The incidence rate of cells affected by shear stress is defined as an area ratio of cells, in which cell death or cell mutation occurs, to normal cells. As illustrated in FIG. 6, the incidence rate of cells affected by shear stress is 0 when it is less than maximum velocity Vmax. Therefore, by discharging the culture liquid while supplying it to the culture dish 3 at the moving linear velocity V which is less than the maximum velocity Vmax, it is possible not to exert influence due to the shear stress on the cultured cells 6. Specific maximum velocity Vmax is about 0.3 m/min when the cultured cells 6 are ES cells of mice.

Moreover, in the present embodiment, because it is configured to continuously supply and discharge the culture liquid to and from the culture dish 3, there is no need to exchange the culture liquid over a long period of time, so that it is possible to obtain a proper cell with a large cell area. Furthermore, each opening of the supply port 4 and the discharge port 5 is large, which is not less than a size such that at least waste product is not clogged when it passes through the opening. Therefore, because clogging does not occur in the filter or the like, also from this point of view, there is no need to exchange the culture liquid over a long period of time, thus, obtaining a proper cell with a large cell area.

When it is ensured that the cultured cells 6 are not affected by the shear stress, the culture liquid is preferably discharged while being supplied to the culture dish 3 so that the velocity is a diffusion velocity V1 or less due to molecular motion of the culture liquid. For the diffusion due to the molecular motion of the culture liquid, unlike artificial diffusion, the flow of the culture liquid does not apply the shear stress to the cultured cells 6. As a result, it is possible to obtain an appropriate cell which is not damaged and has no stress.

Because the diffusion velocity V1 is an extremely small value as compared with the maximum velocity Vmax and each amount of supply and discharge of the culture liquid can be reduced, it is possible to grow the cultured cells 6 at low cost. A minimum moving linear velocity is a velocity at which the cultured cells 6 can obtain necessary nutrients without cell death of the cultured cells 6, and is different for each cultured cell 6. Therefore, it is preferable that the moving linear velocity to be set be the same as or equal to the diffusion velocity. The minimum moving linear velocity is generally about 1/3 of the diffusion velocity.

Here, assuming that an average distance is x[cm] at which molecules move within time t[s], the diffusion phenomenon can be expressed as

$$x=(4Dt)^{(1/2)}$$

Wherein D is a diffusion coefficient, which is about $D=(1 \text{ to } 2)\times 10^{(-5)}[cm^2/s]$ at room temperature.

Therefore, an average distance x at which molecules in the culture liquid move for one minute is 0.4 to 0.7 mm, and the diffusion velocity V1 is 0.4 to 0.7 mm/min.

On the other hand, it is considered that the moving linear velocity V of the culture liquid from the supply port 4 toward the discharge port 5 is the diffusion velocity V1 or less. The cross sectional area from the supply port 4 toward the discharge port 5 in the culture dish 3 is 129 mm^2 when the depth of the culture liquid is 1.5 mm and the width thereof is 86 mm. Therefore, assuming that the flow rate of the culture liquid is set to 42 μL/min, the moving linear velocity V is a value obtained by dividing a flow rate by cross sectional area, which is 0.379 mm/min. In this case, the moving linear velocity V becomes smaller than the diffusion velocity V1.

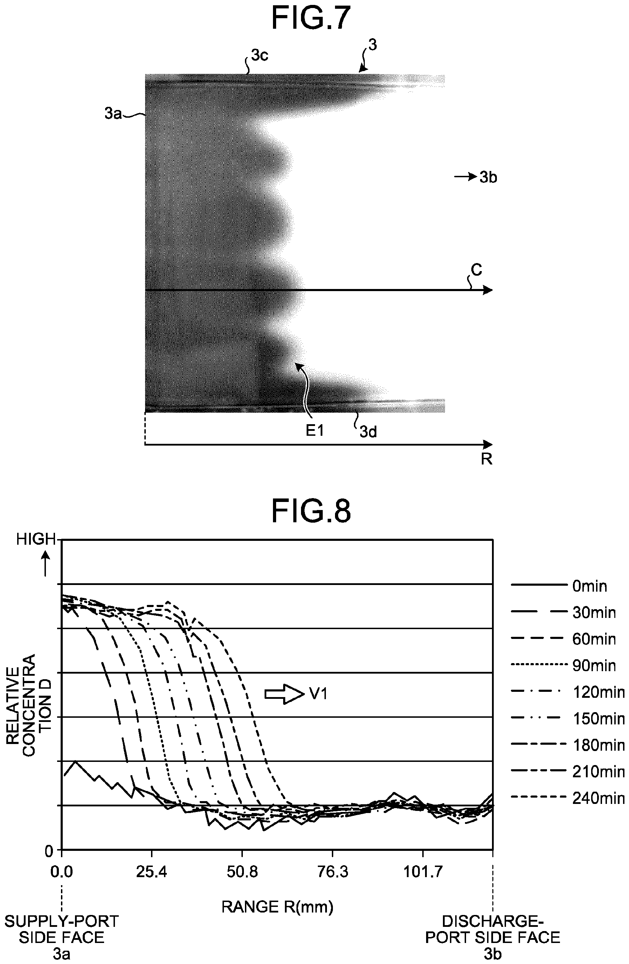

FIG. 7 is a diagram illustrating a state image of the culture liquid flowing from the supply-port side face 3a toward the discharge-port side face 3b when the flow rate is set to the flow rate of the culture liquid. In addition, FIG. 7 is colored by adding methylene blue to the culture liquid to be supplied. As illustrated in FIG. 7, because the supplied culture liquid is supplied from the six supply ports 4, six curves are formed. It is found in each front-edge area E1 of the six curves that the supplied culture liquid diffuses into the existing culture liquid. The front-edge area E1 moves toward the discharge-port side face 3b as time passes. Moreover, the supplied culture liquid becomes dominant on the supply-port side face 3a as time passes. The diffusion velocity is quicker by meniscus on flow-direction side faces 3c and 3d of the culture dish 3 parallel to the direction in which the culture liquid moves toward the discharge-port side face 3b.

The flow rate of the culture liquid to be supplied is controlled so as to be supplied or discharged at a flow rate not exceeding the diffusion state. In other words, the supply amount of the culture liquid is a flow rate that refills the flow rate of the culture liquid diffused from the supply port 4.

FIG. 8 illustrates a change in relative concentration in association with the passage of time on a straight line C illustrated in FIG. 7. A range R is based on the position of the supply-port side face 3a. As illustrated in FIG. 8, the front-edge area E1, in which a relative concentration D is inclined in a bell shape, moves toward the discharge-port side face 3b at the diffusion velocity V1 in association with the passage of time. On the other hand, in the supply-port side face 3a from the front-edge area E1, the front-edge area E1 moves at the moving linear velocity V not more than the diffusion velocity V1, the relative concentration becomes constant, and the supplied culture liquid is dominant.

Flow Control Process of Culture Liquid

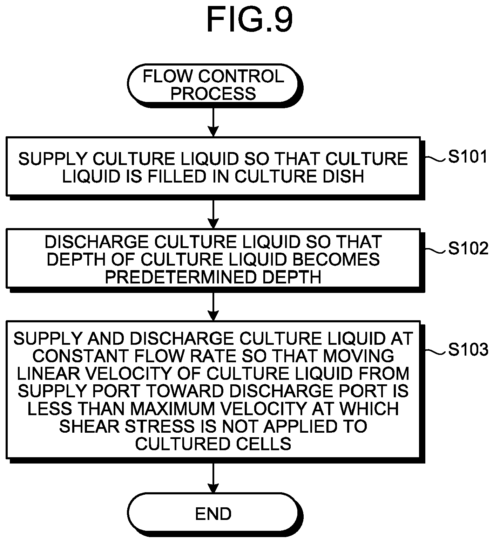

FIG. 9 is a flowchart illustrating a procedure for controlling a flow rate of the culture liquid by the flow rate controller 30. As illustrated in FIG. 9, at first, the cultured cells 6 are stuck to the bottom face of the culture dish 3, and thereafter, the supply-side micro flow rate pump is driven while the discharge-side micro flow rate pump 22 is stopped and the culture liquid is supplied so that the culture liquid is filled in the culture dish 3 (Step S101). Thereafter, the supply-side micro flow rate pump 12 is stopped, the discharge-side micro flow rate pump 22 is driven to discharge the culture liquid, and the depth of the culture liquid is set so as to be a predetermined depth (Step S102). Thereafter, at the predetermined depth, the flow rate is controlled on the supply-side micro flow rate pump 12 and on the discharge-side micro flow rate pump 22 so as to supply and discharge the culture liquid at a constant flow rate so that the moving linear velocity of the culture liquid from the supply port 4 toward the discharge port 5 is less than the maximum velocity at which the shear stress is not applied to the cultured cells 6 or so that the moving linear velocity is preferably equal to or less than the diffusion velocity of the culture liquid (Step S103).

Experimental Result

The cell culture apparatus 1 was used to seed A549 cells (human alveolar basal epithelial carcinoma cells) in the culture dish 3, supply the culture liquid at a diffusion velocity or less and discharge the culture liquid for five days, so that the A549 cells were cultured. Then, it was checked whether shear stress was applied to the cells. Whether the shear stress was applied thereto is understood by checking a phosphorylation state in an NO pathway and a PKC pathway. Experimental results indicate that no eNOS phosphate peptides were identified in the NO pathway. Moreover, KRTS (S73) phosphate peptides were not identified in the PKC pathway, and there was no change in phosphorylation of KRTS (S73). From these results, it can be estimated that no shear stress is applied to the cells.

Arrangement of Supply Ports and Discharge Ports

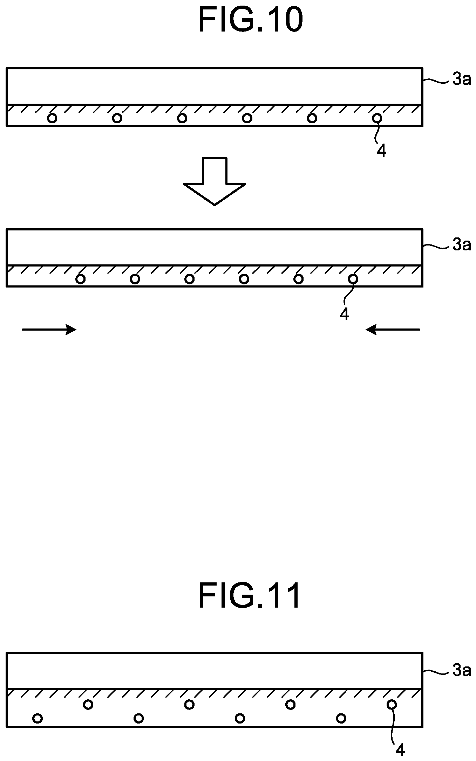

In the embodiment, as illustrated in the upper portion of FIG. 10, the opening positions of the supply ports 4 are arranged at equal intervals linearly in a horizontal direction within a culture liquid level with respect to the supply-port side face 3a, however, as illustrated in the lower portion of FIG. 10, the opening positions of the supply ports 4 may be shifted to the center side. In this case, the influence due to the meniscus is reduced, the front-edge areas E1 can be moved more linearly. The same goes to the opening positions of the discharge ports 5.

When the depth of the culture liquid is deep, as illustrated in FIG. 11, planar arrangement may be adopted so that the opening positions of the supply ports 4 are dispersed also in the depth direction. The same goes to the opening positions of the discharge ports 5.

Suppression of Meniscus Generation

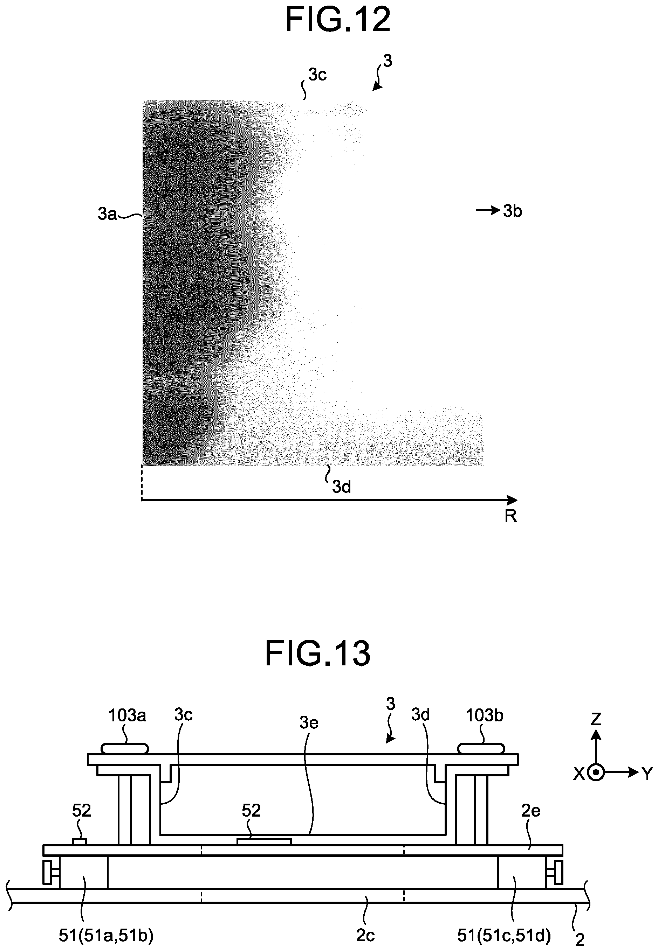

Incidentally, as illustrated in FIG. 7, the moving linear velocity of the culture liquid in the flow-direction side faces 3c and 3d was quick by the meniscus. Therefore, to suppress generation of meniscus, a fluorine-based water repellent agent was applied to the flow-direction side faces 3c and 3d, the supply-port side face 3a, and the discharge-port side face 3b. As a result, even if the opening arrangement of the supply ports 4 was as illustrated in the upper portion of FIG. 10, the moving linear velocity of the culture liquid in the flow-direction side faces 3c and 3d could be suppressed as illustrated in FIG. 12. In other words, by applying the fluorine-based water repellent agent to the flow-direction side faces 3c and 3d, the supply-port side face 3a, and the discharge-port side face 3b, generation of meniscus can be suppressed, and the moving linear velocity of the culture liquid can be made uniform. Because generation of meniscus is only to be suppressed, the fluorine-based water repellent agent only has to be applied to at least the flow-direction side faces 3c and 3d. In addition, the water repellent agent is not limited to a fluorine-based material, and may be a material having water repellency.

Application of the water repellent agent makes smaller the surface free energy of the flow-direction side faces 3c and 3d, the supply-port side face 3a, and the discharge-port side face 3b, than that of the bottom face of the culture dish 3. Therefore, instead of the application of the water repellent agent, the flow-direction side faces 3c and 3d, the supply-port side face 3a, and the discharge-port side face 3b of the culture dish 3, where each has a surface material having the surface free energy smaller than the surface free energy of the bottom face of the culture dish 3, may be used.

Horizontal Adjustment Mechanism of Culture Dish

The front edge of the flow of the culture liquid illustrated in FIG. 12 is oblique to the flow direction. This is because the culture dish 3 is not horizontally disposed, but is inclined to the width direction and the depth of the culture liquid is different in the width direction. Therefore, as illustrated in FIG. 13, a culture-dish substrate 2e to which the culture dish 3 is fixed is provided on the substrate 2, and four horizontal adjustment mechanisms 51 (51a, 51b, 51c, 51d) are provided between the substrate 2 and the culture-dish substrate 2e at positions corresponding to four corners of the culture dish 3. Moreover, two levels 52 for detecting the horizontality between the X direction and the Y direction are fixed to the upper side of the culture-dish substrate 2e. However, if there is any device that can detect the horizontality of the XY plane, the level may be one unit.

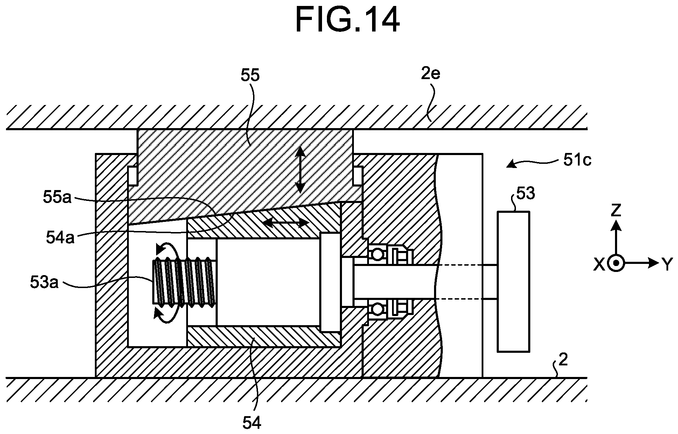

FIG. 14 is a diagram illustrating a configuration of the specific horizontal adjustment mechanism 51c. In the horizontal adjustment mechanism 51c, when an adjustment dial 53 is rotated, a threaded portion 53a formed on the front end side of the adjustment dial 53 rotates. An inclined member 54 screwed to the threaded portion 53a moves in the Y direction according to the rotation of the threaded portion 53a. An elevating member 55 is disposed on the upper portion of the inclined member 54. An inclined portion 54a of the upper portion of the inclined member 54 and an inclined portion 55a of the lower portion of the elevating member 55 slidably abut each other. Therefore, the elevating member 55 moves in a Z direction, which is a height direction, according to the movement of the inclined member 54 in the Y direction. Then, an operator refers to the horizontal state indicated by the levels 52, adjusts the positions of the four horizontal adjustment mechanisms 51a, 51b, 51c, and 51d in the Z direction, and can horizontally adjust the culture-dish substrate 2e. Resultantly, a bottom face 3e of the culture dish 3 can be adjusted horizontally.

When the bottom face 3e of the culture dish 3 is horizontally adjusted by the horizontal adjustment mechanisms 51, the depth of the culture liquid to be supplied to and discharged from the culture dish 3 can be made constant. As a result, the moving linear velocity of the culture liquid in the flow direction (X direction) can be made uniform.

Although the horizontal adjustment mechanisms 51 are manual type, automatic horizontal adjustment mechanisms are preferable. For example, it may be configured so that position images of bubbles indicated by the levels 52 are acquired by an imaging device and a motor-driven adjustment dial is controlled to rotate so that the bubbles move to a horizontal position. By automating the horizontal adjustment, the horizontal state can be always maintained automatically even during culture of the cultured cells 6.

Connection between Liquid Feeding Portion and Liquid Discharging Portion

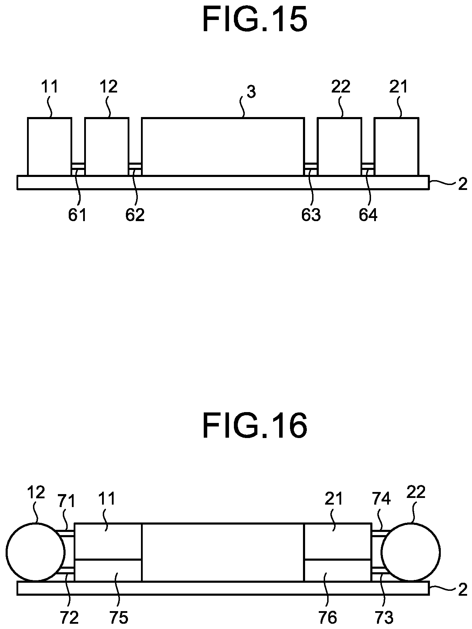

As illustrated in FIG. 15, it may be configured so that a connecting portion 61 between the reservoir tank 11 and the inlet of the supply-side micro flow rate pump 12, a connecting portion 62 between the outlet of the supply-side micro flow rate pump 12 and the supply port 4, a connecting portion 63 between the discharge port 5 and the inlet of the discharge-side micro flow rate pump 22, and a connecting portion 64 between the outlet of the discharge-side micro flow rate pump 22 and the waste liquid tank 21 are directly connected by sockets and plugs with built-in automatic opening/closing valves respectively and can be detachable and one-touch connected.

As illustrated in FIG. 16, it may be configured so that a multistage configuration in which the reservoir tank 11 is provided on an upper side of a supply-side flow path 75 that connects between the supply port 4 and the outlet of the supply-side micro flow rate pump 12 is disposed horizontally in a manner that one end of the multistage configuration provided the reservoir tank 11 therein is directed to the supply port 4 of the culture dish 3, and at the other end of the multistage configuration provided the reservoir tank 11 therein, the inlet of the supply-side micro flow rate pump 12 is detachably connected to the reservoir tank 11 and the outlet of the supply-side micro flow rate pump 12 is detachably connected to the inlet of the supply-side flow path 75.

In other words, it is configured so that a connecting portion 71 between the inlet of the supply-side micro flow rate pump 12 and the reservoir tank 11 and a connecting portion 72 between the outlet of the supply-side micro flow rate pump 12 and the inlet of the supply-side flow path 75 are directly connected by sockets and plugs with built-in automatic opening/closing valves respectively and can be detachable and one-touch connected.

Likewise, it may be configured so that a multistage configuration in which the waste liquid tank 21 is provided on an upper side of a discharge-side flow path 76 that connects between the discharge port 5 and the inlet of the discharge-side micro flow rate pump 22 is disposed horizontally in a manner that one end of the multistage configuration provided the waste liquid tank 21 therein is directed to the discharge port 5 of the culture dish 3, and at the other end of the multistage configuration provided the waste liquid tank 21 therein, the outlet of the discharge-side micro flow rate pump 22 is detachably connected to the waste liquid tank 21 and the inlet of the discharge-side micro flow rate pump 22 is detachably connected to the outlet of the discharge-side flow path 76.

In other words, it is configured so that a connecting portion 74 between the outlet of the discharge-side micro flow rate pump 22 and the waste liquid tank 21 and a connecting portion 73 between the outlet of the discharge-side micro flow rate pump 22 and the outlet of the discharge-side flow path 76 are directly connected by sockets and plugs with built-in automatic opening/closing valves respectively and can be detachable and one-touch connected.

Transportation Form of Cell Culture Apparatus

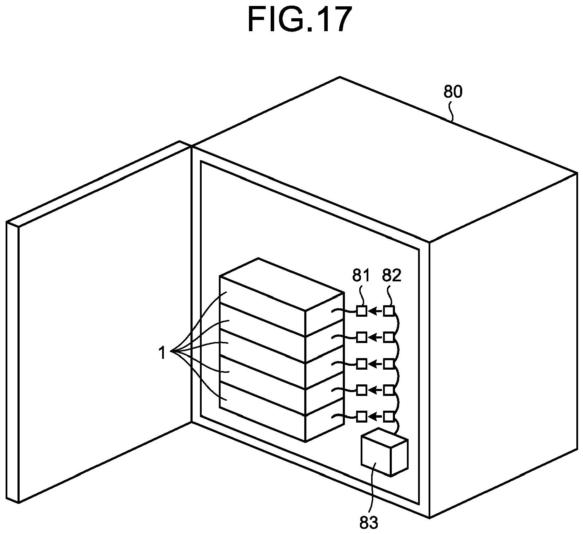

Because the cell culture apparatus is in a flat plate shape, it can be stacked in multiple stages. Therefore, for example, as illustrated in FIG. 17, the cell culture apparatus 1 can be transported by using a transportation box 80, capable of keeping the heat in, in which it is stacked in multiple stages. Power connectors 81 of the cell culture apparatuses 1 are respectively connected to power connectors 82 connected to a battery 83. The power connectors 82 are connected to the battery 83 in such a manner as a string of beads. Therefore, for example, even if the cell culture apparatus 1 at the third stage from the top is removed, only the power connector 81 and the power connector 82 of the removed cell culture apparatus 1 are removed, and the other cell culture apparatuses 1 can continuously culture the cells.

When transporting the cell culture apparatuses 1 in the transportation box 80 or the like, the culture dish 3 is covered with a lid, and it is preferable to fill the space in the culture dish 3 with the culture liquid. This makes it possible to reduce the influence of shear stress on the cultured cells in the culture dish 3 and to perform continuous culture until the cultured cells reach the destination.

Application of Closed Perfusion System Flow Path

In the embodiment, the flexible tube is used for the flow path between the culture dish 3 and the discharge-side micro flow rate pump 22, and the user connects between the flexible tube and the discharge port of the discharge-side micro flow rate pump 22. In a modification of the embodiment, the closed perfusion system flow path in which no opening portions are provided on all the flow paths is formed.

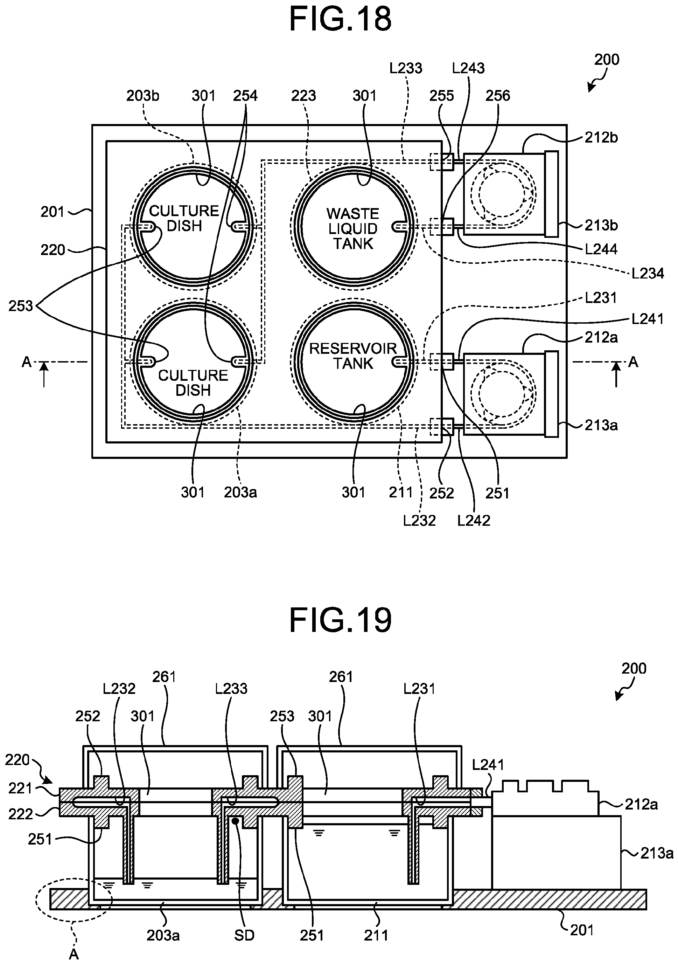

FIG. 18 is a plan view illustrating a configuration of a cell culture apparatus 200 using the closed perfusion system flow path. FIG. 19 is an A-A line cross-sectional view of the cell culture apparatus 200 illustrated in FIG. 18. As illustrated in FIG. 18 and FIG. 19, the cell culture apparatus 200 includes culture dishes 203a and 203b, a reservoir tank 211, a waste liquid tank 223, and motors 213a and 213b, which are provided on a substrate 201. A flow path plate 220 is disposed on the upper side of the culture dishes 203a and 203b, the reservoir tank 211, and the waste liquid tank 223. Supply-side micro flow rate pumps 212a and 212b are disposed on the motors 213a and 213b, respectively. Concave portions are formed in the substrate 201 so that the culture dishes 203a and 203b, the reservoir tank 211, and the waste liquid tank 223 are arranged in the concave portions. An opening is formed in each bottom face of the concave portions.

A flow path is embedded in the flow path plate 220. The flow path plate 220 internally forms a flow path by forming a groove as a flow path on opposing surfaces between an upper plate 221 and a lower plate 222 and joining the opposing surfaces between the upper plate 221 and the lower plate 222. The flow path includes a first flow path L231 between the reservoir tank 211 and the supply-side micro flow rate pump 212a, a second flow path L232 between the supply-side micro flow rate pump 212a and the culture dishes 203a and 203b, a third flow path L233 between the culture dishes 203a and 203b and the discharge-side micro flow rate pump 212b, and a fourth flow path L234 between the discharge-side micro flow rate pump 212b and the waste liquid tank 223.

An inlet 251 between the first flow path L231 and an inlet pipe L241 of the supply-side micro flow rate pump 212a is pin port connected thereto, and an outlet 252 between the second flow path L232 and an outlet pipe L242 of the supply-side micro flow rate pump 212a is pin port connected thereto. An inlet 255 between the third flow path L233 and an inlet pipe L243 of the discharge-side micro flow rate pump 212b is pin port connected thereto, and an outlet 256 between the fourth flow path L234 and an outlet pipe L244 of the discharge-side micro flow rate pump 212b is pin port connected thereto. Thus, the culture liquid within the reservoir tank 211 flows through the closed perfusion system flow path until it is collected in the waste liquid tank 223. Therefore, it is possible to prevent contamination to the culture liquid by workers during assembly of the cell culture apparatus 200. In addition, it is preferable that the pin port connection is previously in a connected state in order to prevent contamination. This eliminates works of the workers forming a flow path at the time of assembly of the cell culture apparatus 200.

Lower engaging portions 251 for being inserted into respective openings of the culture dishes 203a and 203b, the reservoir tank 211, and the waste liquid tank 223 arranged on the substrate 201 to position the flow path plate 220 are formed on the lower side of the flow path plate 220. Moreover, upper engaging portions 252 for preventing displacement of respective lids 261 closing the culture dishes 203a and 203b, the reservoir tank 211, and the waste liquid tank 223 are formed on the upper side of the flow path plate 220. Openings 301 are formed in respective areas of the flow path plate 220 corresponding to respective openings of the culture dishes 203a and 203b, the reservoir tank 211, and the waste liquid tank 223. The flow path plate 220 is provided with nozzles to form respective flow paths into the culture dishes 203a and 203b, the reservoir tank 211, and the waste liquid tank 223.

The cell culture apparatus 200 is configured to provide one supply port 253 and one discharge port 254 in each of the culture dishes 203a and 203b. This is because there may be a case where when a plurality of supply ports branched from one flow path are provided in one culture dish at the time of supplying the culture liquid at a low flow rate, it is difficult to equally branch the flow rate to the respective supply ports.

Moreover, in this modification, liquid level detection sensors SD for detecting a liquid level of the culture liquid in the culture dishes 203a and 203b are provided on the flow path plate 220. A flow rate controller, not illustrated, corresponding to the flow rate controller 30 of FIG. 2 performs flow rate control so that the liquid level detected by the liquid level detection sensor SD is constant. This makes it possible to maintain a constant moving velocity of the culture liquid.

Furthermore, in the modification, an inclination adjustment mechanism for diagonally adjusting the bottom face of each of the culture dishes 203a and 203b is provided at A portion of the substrate 201 illustrated in FIG. 19. The inclination adjustment mechanism can be implemented in the same configuration as that of the horizontal adjustment mechanism 51 illustrated in FIG. 13 and FIG. 14. The inclination by the inclination adjustment mechanism is formed so that the discharge port 254 side in the bottom face of each of the culture dishes 203a and 203b is made lower. This makes it possible to effectively remove dead cells occurring during culture from the discharge port 254. Therefore, it is preferable to match the position of the discharge port 254 with the inclination direction of the inclination adjustment mechanism. The supply port 253 is provided at the position facing the discharge port 254. Therefore, when the culture dish is inclined by the inclination adjustment mechanism, the supply port 253 is provided at a higher position as compared with the discharge port 254. It is also preferable to provide the discharge port of the reservoir tank 211 at a lower position when inclined, similarly to the discharge port 254.

In connecting the pump 212a and the motor 213a, there is a mechanism that the input shaft of the motor 213a can be connected to the pump 212a while rotating. Specifically, the input shaft of the motor 213a can be moved up and down by a spring, and when the position of the fitting hole of the pump 212a and the position of the input shaft of the motor 213a are matched, both of them can be connected by the input shaft being pushed thereinto by the spring. Likewise, in connecting the pump 212b and the motor 213b, there is a mechanism that the input shaft of the motor 213b can be connected to the pump 212b while rotating.

In the embodiment, because the flow rate of the culture liquid to be supplied and discharged is quite low, the consumption of the culture liquid can be reduced even if cells having a large cell area are cultured. In addition, even if a culture period until cells required for recent regenerative medicine are obtained is a long period of time such as about one month, the cost of cell culture can be reduced without consuming a large amount of culture liquid.

Incidentally, the culture liquid is a liquid medium. The liquid medium includes a synthetic medium and a natural medium. The synthetic medium is, unlike the natural medium, a mixture of physiological saline solution (salt solution) with chemical substances such as saccharides and vitamins. The synthetic medium is required to have a composition of a salt solution in which osmotic pressure, pH, ionic composition, and the like are optimum for tissues and cells of specific mammals, reptiles, fish, insects, plants, and the like. In order to find a salt solution in which composition of the salt solution is optimized, the cell culture apparatus according to the present embodiment can be used. In other words, by supplying and discharging the salt solution not including nutrients to and from the cell culture apparatus and observing the survival state of the cells in the cell culture apparatus, the composition of an optimal salt solution can be found. In this case, the cell culture apparatus according to the present embodiment can continuously exclude waste products from the cells for a long time and can remove other factors such as stress and damage for survival of the cells, and can, therefore, determine the composition of the optimal salt solution. When finding an optimal synthetic medium, it is only necessary to further change the composition of various nutrients and observe the survival state of the cells.

In other words, the cell culture apparatus according to the present embodiment supplies a liquid, such as the culture liquid including nutrients required to grow cultured cells and the salt solution not including nutrients required for life support of cultured cells for a short period of time, to the culture dish 3 and discharges the liquid therefrom.

Reference Signs List

    1, 200 CELL CULTURE APPARATUS
    2, 201 SUBSTRATE
    2a, 2b FLANGE
    2c, 2d HOLE
    2e CULTURE-DISH SUBSTRATE
    3, 203a, 203b CULTURE DISH
    3a SUPPLY-PORT SIDE FACE
    3b DISCHARGE-PORT SIDE FACE
    3c, 3d FLOW-DIRECTION SIDE FACE
    3e BOTTOM FACE
    4, 253 SUPPLY PORT
    5, 254 DISCHARGE PORT
    6 CULTURED CELLS
    10 LIQUID FEEDING PORTION
    11, 211 RESERVOIR TANK
    12, 212a, 212b SUPPLY-SIDE MICRO FLOW RATE PUMP
    12a, 22a PUMP GROUP
    12b, 22b SPEED REDUCER
    12c, 22c, 213a, 213b MOTOR
    13, 23 DIAPHRAGM ADJUSTMENT MECHANISM
    20 LIQUID DISCHARGING PORTION
    21, 223 WASTE LIQUID TANK
    22 DISCHARGE-SIDE MICRO FLOW RATE PUMP
    30 FLOW RATE CONTROLLER
    40 TEMPERATURE CONTROLLER
    41 TRANSPARENT CONDUCTIVE FILM HEATER
    42, 44 TEMPERATURE SENSOR

    43 PELTIER ELEMENT
    50 POWER SUPPLY
    51 (51a, 51b, 51c, 51d) HORIZONTAL ADJUSTMENT MECHANISM
    52 LEVEL
    53 ADJUSTMENT DIAL
    53a THREADED PORTION
    54 INCLINED MEMBER
    54a, 55a INCLINED PORTION
    55 ELEVATING MEMBER
    61 to 64, 71 to 74 CONNECTING PORTION
    75 SUPPLY-SIDE FLOW PATH
    76 DISCHARGE-SIDE FLOW PATH
    80 TRANSPORTATION BOX
    81, 82 POWER CONNECTOR
    83 BUTTERY
    103a, 103b, 111a, 111b, 121a, 121b HOLDING MEMBER
    220 FLOW PATH PLATE
    C STRAIGHT LINE
    D RELATIVE CONCENTRATION
    E1 FRONT-EDGE AREA
    L11, L12, L21, L22 FLOW PATH
    L231 FIRST FLOW PATH
    L232 SECOND FLOW PATH
    L233 THIRD FLOW PATH
    L234 FOURTH FLOW PATH
    P1, P2 PRESSURE
    R RANGE
    SD LIQUID LEVEL DETECTION SENSOR
    V MOVING LINEAR VELOCITY
    V1 DIFFUSION VELOCITY
    Vmax MAXIMUM VELOCITY

**Claims**

1.  A cell culture method for arranging cultured cells in a culture dish and continuously culturing the cultured cells by supplying a liquid required to grow or maintain the cultured cells to the culture dish and discharging the liquid from the culture dish, the cell culture method comprising:

    providing a supply port of the liquid at one end of the culture dish and providing a discharge port of the liquid at other end of the culture dish so as to sandwich the cultured cells between the supply port and the discharge port, and discharging the liquid while supplying the liquid to the culture dish so that a moving linear velocity of the liquid from the supply port toward the discharge port is less than a maximum velocity at which shear stress is not applied to the cultured cells.

2.  The cell culture method, wherein the moving linear velocity of the liquid is equal to or less than a diffusion velocity due to molecular motion of the liquid.

3. A cell culture apparatus configured to arrange cultured cells in a culture dish and continuously culture the cultured cells by supplying a liquid required to grow or maintain the cultured cells to the culture dish and discharging the liquid from the culture dish, comprising:

a supply port of the liquid provided at one end of the culture dish;

a discharge port for the culture dish provided at other end of the culture dish so as to sandwich the cultured cells between the supply port and the discharge port,

a reservoir tank configured to store the liquid to be supplied to the culture dish;

a waste liquid tank configured to store the liquid to be discharged from the culture dish;

a supply-side micro flow rate pump configured to supply the liquid in the reservoir tank to the culture dish through the supply port;

a discharge-side micro flow rate pump configured to discharge the liquid from the culture dish through the discharge port; and

a flow rate controller configured to perform flow rate control of the supply-side micro flow rate pump and the discharge-side micro flow rate pump so that a moving linear velocity of the liquid from the supply port toward the discharge port is less than a maximum velocity at which shear stress is not applied to the cultured cells.

4. The cell culture apparatus according to claim 3, wherein the moving linear velocity of the liquid is equal to or less than a diffusion velocity due to molecular motion of the liquid.

5. The cell culture apparatus according to claim 3 or 4, wherein a side face of the culture dish has surface free energy smaller than surface free energy of a bottom face of the culture dish.

6. The cell culture apparatus according to any one of claims 3 to 5, further comprising:

a horizontal adjustment mechanism configured to adjust the bottom face of the culture dish horizontally.

7. The cell culture apparatus according to any one of claims 3 to 6, further comprising:

an inclination adjustment mechanism configured to adjust the bottom face of the culture dish diagonally.

8. The cell culture apparatus according to any one of claims 3 to 7, wherein
the supply port comprises a plurality of supply ports which are discretely arranged in linear order, and the discharge port comprises a plurality of discharge ports which are discretely arranged in linear order.

9. The cell culture apparatus according to any one of claims 3 to 7, wherein
an outlet of the supply-side micro flow rate pump and the supply port are connected by a flexible tube, and the discharge port and an inlet of the discharge-side micro flow rate pump are connected by a flexible tube, and
a diaphragm adjustment mechanism configured to adjust an opening of the flexible tube connected between the outlet of the supply-side micro flow rate pump and the supply port is provided between the supply-side micro flow rate pump and the supply port, and a diaphragm adjustment mechanism configured to adjust an opening of the flexible tube connected between the discharge port and the inlet of the discharge-side micro flow rate pump is provided between the discharge-side micro flow rate pump and the discharge port.

10. The cell culture apparatus according to any one of claims 3 to 7, wherein
the reservoir tank and an inlet of the supply-side micro flow rate pump are detachably and directly connected to each other,
an outlet of the supply-side micro flow rate pump and the supply port are detachably and directly connected to each other,
the discharge port and an inlet of the discharge-side micro flow rate pump are detachably and directly connected to each other, and
an outlet of the discharge-side micro flow rate pump and the waste liquid tank are detachably and directly connected to each other.

11. The cell culture apparatus according to claim 10, wherein
a multistage configuration in which the reservoir tank is provided on an upper side of a supply-side flow path that connects between the supply port and the outlet of the supply-side micro flow rate pump is disposed horizontally in a manner that one end of the multistage configuration provided the reservoir tank therein is directed to the supply port of the culture dish, and at other end of the multistage configuration provided the reservoir tank therein, the inlet of the supply-side micro flow rate pump is detachably connected to the reservoir tank and the outlet of the supply-side micro flow rate pump is detachably connected to an inlet of the supply-side flow path, and
a multistage configuration in which the waste liquid tank is provided on an upper side of a discharge-side flow path that connects between the discharge port and the inlet of the discharge-side micro flow rate pump is disposed horizontally in a manner that one end of the multistage configuration provided the waste liquid tank therein is directed to the discharge port of the culture dish, and at the other end of the multistage configuration provided the waste liquid tank therein, the outlet of the discharge-side micro flow rate pump is detachably connected to the waste liquid tank and the inlet of the discharge-side micro flow rate pump is detachably connected to an outlet of the discharge-side flow path.

12. The cell culture apparatus according to any one of claims 3 to 7, further comprising:

a flow path plate in which a first flow path between the reservoir tank and the supply-side micro flow rate pump, a second flow path between the supply-side micro flow rate pump and the culture dish, a third flow path between the culture dish and the discharge-side micro flow rate pump, and a fourth flow path between the discharge-side micro flow rate pump and the waste liquid tank are embedded, the flow path plate being disposed on an upper side of the reservoir tank, the culture dish, and the waste liquid tank, wherein
the first flow path and an inlet of the supply-side micro flow rate pump are pin port connected to each other,
the second flow path and an outlet of the supply-side micro flow rate pump are pin port connected to each other,
the third flow path and an inlet of the discharge-side micro flow rate pump are pin port connected to each other, and
the fourth flow path and an outlet pipe of the supply-side micro flow rate pump are pin port connected to each other.

13. The cell culture apparatus according to any one of claims 3 to 12, further comprising:

a liquid level detection sensor configured to detect a liquid level in the culture dish, wherein
the flow rate controller is configured to perform flow rate control so that the liquid level detected by the liquid level detection sensor becomes constant.

14. The cell culture apparatus according to any one of claims 3 to 13, wherein
a supply-side drive source for driving the supply-side micro flow rate pump is configured to be detachably attached to the supply-side micro flow rate pump, and a discharge-side drive source for driving the discharge-side micro flow rate pump is configured to be detachably attached to the discharge-side micro flow rate pump.

15. The cell culture apparatus according to any one of claims 3 to 14, further comprising:

a substrate configured to arrange at least the culture dish, wherein
the substrate has a hole or a colorless and transparent concave portion in an area of the substrate where a culture state of the cultured cells is observed, the concave portion having an opening formed in the substrate on a side opposite to a side of the substrate on which the culture dish is arranged.

16. The cell culture apparatus according to any one of claims 3 to 15, further comprising:

a transparent conductive film heater provided at a bottom face of the culture dish;
a temperature sensor configured to detect a temperature of the liquid in the culture dish; and
a temperature controller configured to perform control to keep the temperature of the liquid in the culture dish within a predetermined temperature range by energizing the transparent conductive film heater based on detection result of the temperature sensor.

EP 3 235 902 A1

# FIG.1

# FIG.2

EP 3 235 902 A1

# FIG.3

# FIG.4

# FIG.5

# FIG.6

# FIG.7

# FIG.8

# FIG.9

```
        ( FLOW CONTROL )
        (    PROCESS    )
               │
               ▼
┌──────────────────────────────────┐
│ SUPPLY CULTURE LIQUID SO THAT     │  ⌐S101
│ CULTURE LIQUID IS FILLED IN       │
│ CULTURE DISH                      │
└──────────────────────────────────┘
               │
               ▼
┌──────────────────────────────────┐
│ DISCHARGE CULTURE LIQUID SO THAT  │  ⌐S102
│ DEPTH OF CULTURE LIQUID BECOMES   │
│ PREDETERMINED DEPTH               │
└──────────────────────────────────┘
               │
               ▼
┌──────────────────────────────────┐
│ SUPPLY AND DISCHARGE CULTURE      │  ⌐S103
│ LIQUID AT CONSTANT FLOW RATE SO   │
│ THAT MOVING LINEAR VELOCITY OF    │
│ CULTURE LIQUID FROM SUPPLY PORT   │
│ TOWARD DISCHARGE PORT IS LESS     │
│ THAN MAXIMUM VELOCITY AT WHICH    │
│ SHEAR STRESS IS NOT APPLIED TO    │
│ CULTURED CELLS                    │
└──────────────────────────────────┘
               │
               ▼
          (   END   )
```

# FIG.10

# FIG.11

# FIG.12

3c
3
3a
→3b
3d
R

# FIG.13

103a    3c    3e    3    3d    103b    Z
52    52    2e    X⊙→Y
51(51a,51b)    2c    51(51c,51d)    2

# FIG.14

# FIG.15

11    12    3    22    21

61    62    63    64    2

# FIG.16

12  71  11    21  74  22

72  75    76  73  2

# FIG.17

# FIG.18

# FIG.19

# INTERNATIONAL SEARCH REPORT

| International application No. |
|---|
| PCT/JP2015/084698 |

**A. CLASSIFICATION OF SUBJECT MATTER**
*C12N5/071*(2010.01)i, *C12M1/00*(2006.01)i, *C12M3/00*(2006.01)i

According to International Patent Classification (IPC) or to both national classification and IPC

**B. FIELDS SEARCHED**

Minimum documentation searched (classification system followed by classification symbols)
C12N5/071, C12M1/00, C12M3/00

Documentation searched other than minimum documentation to the extent that such documents are included in the fields searched
　　Jitsuyo Shinan Koho　　　　1922-1996　　Jitsuyo Shinan Toroku Koho　1996-2016
　　Kokai Jitsuyo Shinan Koho　1971-2016　　Toroku Jitsuyo Shinan Koho　1994-2016

Electronic data base consulted during the international search (name of data base and, where practicable, search terms used)
　　JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2007-020493 A　(Takagi Industrial Co., Ltd.), 01 February 2007 (01.02.2007), claims; paragraphs [0001], [0019], [0058] to [0063]; fig. 4, 20 & US 2007/0020750 A1 claims; paragraphs [0003], [0022], [0083] to [0088]; fig. 4, 20 & EP 1746154 A1　　　　& KR 10-2007-0011068 A & CN 1900265 A | 1-16 |
| A | WO 2013/152316 A1　(CORSOLUTIONS LLC), 10 October 2013 (10.10.2013), entire text & JP 2015-513980 A　　　& US 2015/0119898 A1 & EP 2833960 A　　　　& KR 10-2015-0014445 A | 1-16 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 　　24 February 2016 (24.02.16) | 　　08 March 2016 (08.03.16) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (January 2015)

**EP 3 235 902 A1**

**INTERNATIONAL SEARCH REPORT**

| International application No. |
|---|
| PCT/JP2015/084698 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2013/011962 A1  (Japan Science and Technology Agency), 24 January 2013 (24.01.2013), entire text & JP 5231684 B           & US 9139807 B2 & EP 2733199 A1          & CN 103649302 A & KR 10-2014-0019475 A | 1-16 |
| A | JP 2012-506257 A  (Biovest International, Inc.), 15 March 2012 (15.03.2012), entire text & US 2011/0212493 A1    & WO 2010/048417 A2 & EP 2346984 A1 | 1-16 |
| A | JP 4-063584 A  (Fuji Photo Film Co., Ltd.), 28 February 1992 (28.02.1992), entire text & US 5376548 A | 1-16 |
| A | KE LIU, et al, Cell Culture Chip Using Low-Shear Mass Transport, Langmuir, 2008, Vol.24, p.5955-5960 | 1-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012170366 A
- JP 2012090632 A